# EUROPÄISCHE PATENTSCHRIFT

(11) **EP 1 094 794 B1**
(45) Veröffentlichungstag und Bekanntmachung des Hinweises auf die Patenterteilung: **17.12.2003**
(21) Anmeldenummer: 99932794.3
(22) Anmeldetag: 02.07.1999
(51) Int. Cl.: A61K 9/70, B05B 5/053, H02N 13/00

(54) **TROCKENKOPIERPROZESS ZUR HERSTELLUNG FLÄCHIGER, EINZELDOSIERTER WIRKSTOFFZUBEREITUNGEN**
DRY-COPYING METHOD FOR PRODUCING FLAT, INDIVIDUALLY DOSED PREPARATIONS OF ACTIVE AGENTS
PROCEDE DE COPIAGE A SEC POUR LA FABRICATION DE PREPARATIONS DE SUBSTANCE ACTIVE, EN SURFACE PLANE ET EN UN SEUL DOSAGE

(30) Priorität: 09.07.1998 DE 19830650
(43) Veröffentlichungstag der Anmeldung: 02.05.2001
(73) Patentinhaber: LTS Lohmann Therapie-Systeme AG, 56626 Andernach (DE)
(72) Erfinder: MÜLLER, Walter, D-56564 Neuwied (DE)
(74) Vertreter: Flaccus, Rolf-Dieter, Dr.
(86) Internationale Anmeldenummer: EP9904612
(87) Internationale Veröffentlichungsnummer: WO00002533

(56) Entgegenhaltungen:
- WO-A-92/14451
- WO-A-96/35516
- WO-A-96/39257
- WO-A-97/38480
- WO-A-98/20861

## Beschreibung

Unter einzeldosierten Wirkstoffzubereitungen werden Zubereitungen verstanden, die eine vorgegebene Menge an aktive Substanz in abgeteilten und einzeln handhabbaren Einheiten der Zubereitung enthalten.

Unter einem Wirkstoff im Sinne dieser Erfindung kann dabei z.B. ein Arzneistoff, ein Insektizid, ein Pestizid, ein Reagenz etc. verstanden werden.

Wird unter einer aktiven Substanz beispielhaft ein medizinischer Wirkstoff verstanden, sind solche einzeldosierten Arzneiformen etwa Tabletten, Kapseln, Suppositorien oder transdermale therapeutische Systeme (TTS).

Nichteinzeldosierte Arzneiformen wären z.B. Sprays, Säfte, Salben und Tropfen, bei denen die eigentliche Dosierung erst unmittelbar vor Gebrauch erfolgt.

Bei einzeln dosierten Wirkstoffzubereitungen ist dabei der Wirkstoff in abgemessener Menge zusammen mit eventuell notwendigen Hilfsstoffen zu der für die Anwendung fertiger Wirkstoffzubereitungen zu verarbeiten. Diese Technik kann bei der Herstellung von Tabletten und Kapseln als ausgereift betrachtet werden. Moderne Tablettenpressen und Kapselfüllmaschinen garantieren eine hohe Produktionsgeschwindigkeit und einer sehr genaue Dosierung in einem Bereich von 95-105 % des Sollwertes.

Das Dokument WO-A-98 20861 zeigt ein Verfahren zur Dosierrung von pulverförmigen wirkstoffen auf Tabletten oder flächen-förmigen Substraten, darunter auch TTS.

Transdermale therapeutische Systeme sind noch eine relative junge Arzneiform, die extern in Form eines Pflasters auf der Haut angewendet wird und den Wirkstoff durch die Haut an den Organismus abgibt. Zur Herstellung dieser TTS werden spezielle Dosiertechniken angewendet und weitere Innovationen bezüglich neuer Dosiertechniken sind möglich.

Bei einer Ausführung dieser TTS ist der Wirkstoff direkt in dem Kleber enthalten und wird bei der Herstellung zusammen mit dem Kleber flächenförmig auf eine Folie dosiert.

Damit sind TTS die einzige Arzneiform, bei der es darauf ankommt, einen medizinischen Wirkstoff auf eine vorgegeene Fläche in vorgegebener Menge zu dosieren. Denkt man an nichtmedizinische Wirkstoffe, wie z.B. Insektizide, Pestizide oder Reagenzien, so sind flächige Zubereitungen in Form von imprägnierten Papieren, Folien oder Kartons schon länger bekannt. Allerdings werden für diese Anwendungen an die Genauigkeit der Dosierung in der Fläche keine **großen** Anforderungen gestellt.

Der vorliegenden Erfindung liegt die Aufgabe zugrunde, ein Verfahren zur Dosierung von pulverförmigen Wirkstoffen auf eine vorgegebene Fläche bereitzustellen.

Diese Aufgabe wird unter Verwendung der aus dem Trockenkopierprozeß bekannten Techniken gelöst durch ein Verfahren gemäß den Merkmalen des Hauptanspruchs 1.

Es hat sich gezeigt, daß diese Technik in der Lage ist, pulverförmige Wirkstoffe mit einer auch für Arzneimittel genügenden Genauigkeit auf eine Fläche zu dosieren. Unmittelbar einsichtig ist dies bei Farbkopierern, bei denen jede Farbe getrennt dosiert wird und größere Ungenauigkeiten zu einer Farbverfälschung führen würden.

Der Trockenkopierprozeß oder die Xerographie funktioniert in der nachfolgend beschriebenen Weise.

Eine mit einem photoleitfähigen Material beschichtete Walze wird mit einer positiven elektrischen Ladung versehen und über eine geeignete Optik mit der zu kopierenden Vorlage belichtet. Durch diesen Vorgang wird die Ladung jeweils dort zumindest teilweise entfernt, wo durch die Belichtung die Leitfähigkeit des photoleitfähigen Materials ansteigt.

In einem nächsten Schritt werden negativ geladene Farbpartikel (Toner) auf die Walze aufgebracht, die allerdings nur auf den noch geladenen Flächen der Walze in von der Größe der Ladung abhängigen Menge haften bleiben und dort ein sichtbares Bild entstehen lassen.

Anschließend werden die Farbpartikel (Toner) auf ein positiv geladenes Papier oder Folie übertragen und dort durch eine Hitzebehandlung fixiert.

Eine moderne Variante des Verfahrens wird bei den sogenannten Laserdruckern eingesetzt. Hier wird ohne Vorlage die Walze direkt mit einem Laserstrahl belichtet und damit das Bild computergesteuert direkt auf die Walze geschrieben. Ansonsten wird bei Laserprintern die gleiche Technik benutzt wie bei Trockenkopierern.

Die vorliegende Erfindung basiert auf der Verwendung dieser Prozesse, um statt der Farbpigmente Wirkstoffpartikel auf Papier bzw. allgemein Folien zu übertragen.

In Versuchen mit einem normalen Schwarzweißkopierer wurde ermittelt, welche Mengen an Toner auf Folie mit welcher Reproduzierbarkeit übertragen werden können.

Bei der verwendeten Folie handelt es sich um handelsübliche große Overhead-Folien aus Polyester im Format DIN A4. Als Vorlage wurde ein tiefschwarzer Bogen Papier verwendet und der stärkste Schwärzungsgrad für die Kopie gewählt.

Die Ergebnisse sind in folgender Tabelle festgehalten.

**Tabelle 1**

| versuchsnummer | Auftrag Toner (mg/cm²) |
|---|---|
| 1 | 0,609 |
| 2 | 0,601 |
| 3 | 0,640 |
| 4 | 0,603 |
| 5 | 0,603 |
| 6 | 0,604 |
| Mittelwert | 0,610 |
| rel. Fehler (%) | 2,4 |

Die Ergebnisse zeigen, daß zwar nur eine vergleichsweise kleine Menge an Toner übertragen wurde, dies jedoch mit einer auch für medizinische Anwendungen geeigneten Genauigkeit.

In einem weiteren Versuch wurde nun ermittelt, ob die Menge durch Mehrfachdosierung erhöht werden kann.

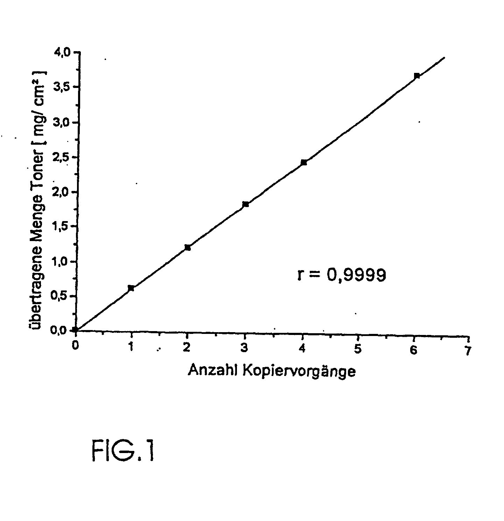

Die Ergebnisse sind in FIG.1 dargestellt und zeigen, daß die übertragene Menge Toner proportional der Anzahl der Kopiervorgänge ist.

Dieses Ergebnis zeigt, daß die in einem Kopierprozeß übertragene Menge zwar klein ist, dieser Nachteil jedoch durch mehrfaches Kopieren überwunden werden kann, ohne daß die Genauigkeit der Dosierung darunter leidet.

Die Menge an übertragenem Toner bzw. Wirkstoff hängt von dem Ladungszustand der Kopierwalze ab und kann durch eine stärkere Aufladung über das bei Kopiergeräten übliche Maß gesteigert werden.

Wenn der Wirkstoff ohne spezielles Muster dosiert wird, kann auf den Belichtungsschnitt ganz verzichtet werden. In diesem Fall braucht die Kopierwalze nicht aus einem photoleitfähigen Material zu bestehen.

Soll die Dosierung in einem Muster erfolgen, ist der Belichtungsschnitt und die Verwendung einer mit einem photoleifähigen Material, z.B. Selen, beschichteten Kopierwalze notwendig. Die Belichtung kann dabei über ein Vorlage oder aber auch coputergesteuert über einen Laser erfolgen. Die größte variabilität ist natürlich mit der computergesteuerten Laserbelichtung gegeben.

Es ist davon auszugehen, daß die meisten Wirkstoffe farblos sind. Unter diesem Aspekt ist es sinnvoll, der pulverförmigen Wirk- und Hilfsstoffmischung einen Indikatorfarbstoff beizumischen. Dieser Farbstoff erlaubt dann nach entsprechender Eichung, mit optischen Methoden direkt die Menge an übertragenem Wirkstoff zu messen und eventuellen Abweichungen vom Sollwert automatisch entgegenzusteuern.

Das flächige Substrat, auf das der Wirkstoff übertragen wird, kann dabei im Prinzip aus beliebigen Materialien bestehen, solange es flexibel und geeignet ist, den Fixiervorgang zu überstehen. Dabei ist denkbar, daß das Material in Form von Blättern eingesetzt wird oder von einer Rolle kommt. Dieses Material wird dann im Zuge der Weiterverarbeitung in kleinere Teile geschnitten und damit in die einzeldosierte Wirkstoffzubereitung überführt. Alternativ kann das Material auch perforiert sein, wobei dann die Dosierung durch die Perforierung vorgegeben ist.

## Patentansprüche

1. Verfahren zur Dosierung von pulverförmigen Wirkstoff auf eine vorgegebene Fläche, **dadurch gekennzeichnet, daß**
- der Wirkstoff als elektrisch geladenes Pulver auf eine entgegengesetzt geladene Walze übertragen,
- der auf die Walze übertragene Wirkstoff auf ein flächenförmiges Substrat mit zum Wirkstoff entgegengesetzter elektrischer Ladung transferiert,
- der auf das Substrat übertragene Wirkstoff mittels einer Hitzebehandlung fixiert wird.

2. Verfahren gemäß Anspruch 1, **dadurch gekennzeichnet, daß** zusammen mit dem Wirkstoff ebenfalls pulverförmige Hilfsstoffe dosiert werden.

3. Verfahren gemäß Anspruch 2, **dadurch gekennzeichnet, daß** zumindest ein Hilfsstoff als Fixiermittel fungiert.

4. Verfahren gemäß Anspruch 2, **dadurch gekennzeichnet, daß** ein Hilfsstoff farbig ist und zur optischen Ermittlung der Wirkstoffmenge dient.

5. Verfahren gemäß Anspruch 1 bis 4, **dadurch gekennzeichnet, daß** die Walze mit einem photoleitenden Material überzogen ist.

6. Verfahren gemäß Anspruch 1, **dadurch gekennzeichnet, daß** die Ladung der Walze und damit die Menge und/oder das Muster der Wirkstoffübertragung durch eine Belichtung der geladenen Walze beeinflußt wird.

7. Verfahren gemäß Anspruch 6, **dadurch gekennzeichnet, daß** die Belichtung der Walze über einen computergesteuerten Laser oder konventionell über die optische Abbildung einer Vorlage geschieht.

## Claims

1. Method for metering active ingredient in powder form onto a predetermined area, **characterized in that**
- the active ingredient is transferred as electrically charged powder to a roll with the opposite charge,
- the active ingredient transferred to the roll is transferred to a two-dimensional substrate with an electric charge opposite to the active ingredient,
- the active ingredient transferred to the substrate is fixed by means of a heat treatment.

2. Method according to Claim 1, **characterized in that** inactive ingredients likewise in powder form are metered together with the active ingredient.

3. Method according to Claim 2, **characterized in that** at least one inactive ingredient acts as fixative.

4. Method according to Claim 2, **characterized in that** one inactive ingredient is coloured and is used for optical determination of the amount of active ingredient.

5. Method according to Claim 1 to 4, **characterized in that** the roll is coated with a photoconducting material.

6. Method according to Claim 1, **characterized in that** the charge on the roll and thus the amount and/or the pattern of active ingredient transfer is influenced by exposure of the loaded roll.

7. Method according to Claim 6, **characterized in that** the exposure of the roll takes place via a computer-controlled laser or conventionally via the optical imaging of an original.

## Revendications

1. Procédé pour le dosage d'une substance active pulvérulente sur une surface donnée, **caractérisé en ce que**
- la substance active est appliquée comme poudre chargée électriquement sur un cylindre à charge inverse,
- la substance active appliquée sur le cylindre est transférée sur un substrat planiforme avec une charge électrique inverse à la substance active,
- la substance active appliquée sur le substrat est fixée par un traitement thermique.

2. Procédé selon la revendication 1, **caractérisé en ce que** des adjuvants pulvérulents sont également ajoutés avec la substance active.

3. Procédé selon la revendication 2, **caractérisé en ce qu'**au moins un adjuvant fait fonction de fixatif.

4. Procédé selon la revendication 2, **caractérisé en ce qu'**un adjuvant est coloré et sert à déterminer optiquement la quantité de substance active.

5. Procédé selon les revendications 1 à 4, **caractérisé en ce que** le cylindre est recouvert d'une matière photoconductrice.

6. Procédé selon la revendication 1, **caractérisé en ce que** le chargement du cylindre, et ainsi la quantité et/ou le dessin de l'application de substance active, est influencé par une exposition à la lumière du cylindre chargé.

7. Procédé selon la revendication 6, **caractérisé en ce que** l'exposition à la lumière du cylindre s'effectue par un laser commandé par ordinateur ou de manière conventionnelle par la représentation optique d'un modèle.
